# EUROPEAN PATENT APPLICATION

(11) **EP 3 984 576 A2**
(43) Date of publication of application: **20.04.2022**
(21) Application number: 20826462.2
(22) Date of filing: 17.06.2020
(51) Int. Cl.: A61M 5/315

(54) **PLUNGER CAPABLE OF MOVING BACK AND FORTH AND MEDICINE INJECTION DEVICE INCLUDING SAME**

(30) Priority: 17.06.2019 KR 20190071236
(71) Applicant: Noh, Wook Rea, Suwon-si, Gyeonggi-do 16305 (KR)
(72) Inventor: Noh, Wook Rea, Suwon-si, Gyeonggi-do 16305 (KR)
(74) Representative: Isarpatent
(86) International application number: PCT/KR2020/007801
(87) International publication number: WO 2020/256386

(57) **Abstract**

The present invention relates to a plunger capable of moving back and forth and a medicine injection device including same, in which, by manually or automatically moving the plunger backwards, user convenience may be provided. In addition, according to the present invention, when a rotation unit (20) is no longer rotated, first flat portions (151-1, 151-2) of a first adjusting portion (150-1) and a second adjusting portion (150-2) are slid in an inclined direction, that is, in a direction from a second housing (120) to the rotation unit (20), respectively along a first inclined portion (121) and a second inclined portion (122), so that the first adjusting portion (150-1) and the second adjusting portion (150-2) are spaced apart from each other, and thus a pushrod unit (10) can be moved in a direction of the rotation unit (20), that is, backwards.

## Description

### TECHNICAL FIELD

The present disclosure relates to a plunger movable back and forth and a medicine injection device including the same and, more particularly, to a plunger movable back and forth, wherein the plunger may be manually or automatically moved backward to provide user convenience, and a medicine injection device including the same.

### BACKGROUND ART

A medicine injection device is a self-contained device allowing a user requiring self-management while having no formal medical training to inject a medicine or other injectable agent into his or her own body.

Such medicine injection devices are categorized as a pump type attachable to a belt and a pen type that may be carried in a garment pouch, a bag, or the like.

The pump type medicine injection device is attached to the body of a user, such that the user may be freely injected with a medicine in any place at any time while activities of the user are not restricted. The user uses the pump type medicine injection device by manually adjusting the dose of the medicine.

The pen type medicine injection device may be operated in a simple manner, does not feel like a syringe, and may simply inject a medicine. The pen type medicine injection device is used when the user moves a lot.

In addition, the medicine injection device generally includes a needle through which a medicine is injected into a human body, a cartridge containing the medicine, a housing accommodating the cartridge and allowing a plunger to be introduced thereinto, and the plunger configured to be introduced into the housing to push the medicine, thereby assisting in the injection of the medicine.

In general, the plunger used in the medicine injection device has a rubber member coupled to one end of the plunger such that the plunger is used to compress the medicine or transfer pressure through combination of the housing together with the piston.

In addition, the plunger includes a gear provided on the other end thereof, the gear being connected to a motor. Thus, the plunger is automatically driven to move back and forth.

When the medicine within the cartridge of the medicine injection device is exhausted, the plunger is moved to the original position by driving the motor in order to replace the cartridge.

However, the method of moving the plunger backward using the motor may take a significant amount of time, and thus there are problems in that the waiting time of the user may be increased, the motor may be noisy, or the lifespan of the motor may be shortened.

Background-art technologies for overcoming such problems include Japanese Patent No. 5567073 (hereinafter, referred to as Document 1), Japanese Patent No. 5453394 (hereinafter, referred to as Document 2), Japanese Patent No. 5712200 (hereinafter, referred to as Document 3), and Korean Patent No. 10-1541662 (hereinafter, referred to as Document 4).

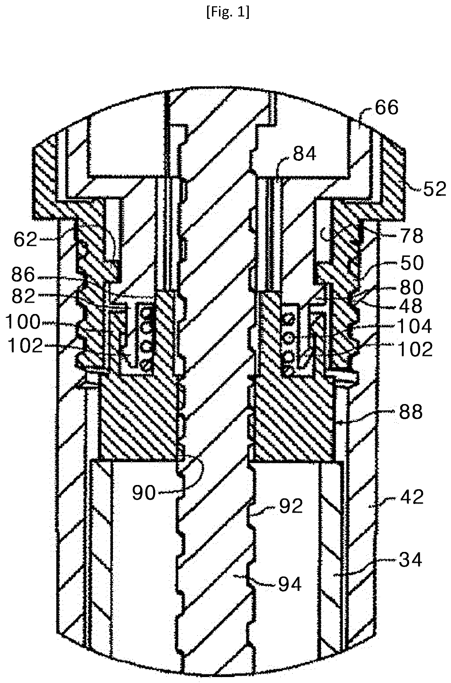

Document 1 relates to a syringe. As illustrated in FIG. 1, during cartridge replacement, rows of teeth 84 and 86 arranged in a first direction of the axis are pressed and separated from each other by a spring 104, thereby allowing a nut member 88 to rotate. Thus, a user may slide a plunger 94 in the direction of the original position.

Since the syringe of Document 1 uses the spring, there are problems in that the elasticity of the spring may be reduced due to frequent use, and the spring may be rusted due to exposure to moisture.

Document 2 relates to a medicine injection device including a dose setting means and a drive device by which a dose of medicine from a medicine cartridge may be repeatedly administered.

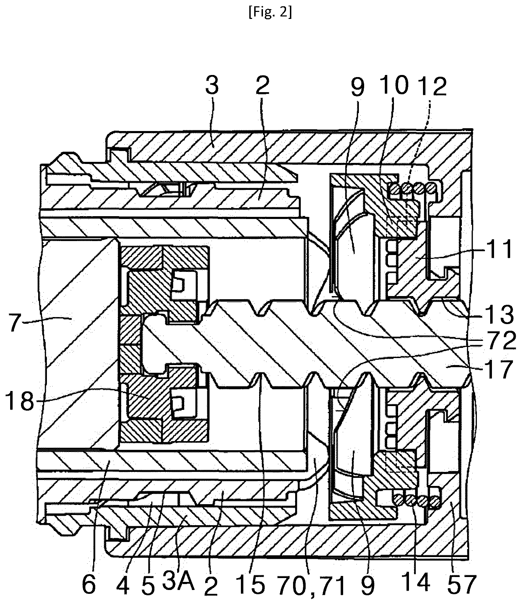

As illustrated in FIG. 2, in the medicine injection device of Document 2, a nut means 11 is freely rotatable, so that a plunger 17 may be rewound to the original position. There is a problem in that a connection between a cartridge holder 2 and a housing 3 should be released.

Document 3 relates to a medicine delivery device including a planetary gearing configured to increase power required to deliver a medicine from the device.

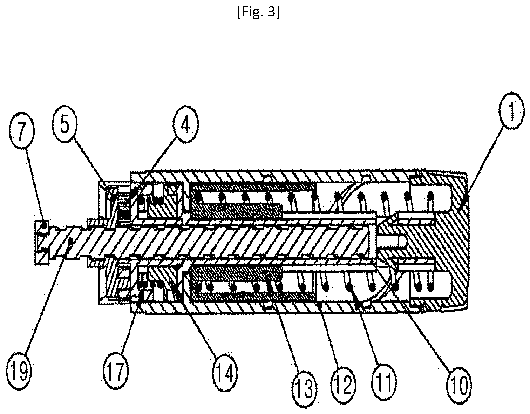

In the device of Document 3, as illustrated in FIG. 3, the plunger is driven using the planetary gearing included inside a plunger housing. Thus, it is difficult for a user to manually move the plunger to the original position.

Document 4 relates to a refill module for an injection device. The injection device includes a housing, a syringe sub-assembly, a carrier, and at least one latching element.

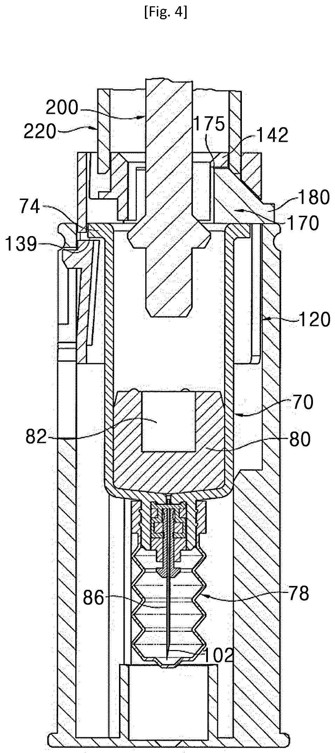

In the injection device of Document 4, as illustrated in FIG. 4, when a tab 180 is in contact with the inside wall of the housing, the tab 180 is not aligned with a housing opening 48. Consequently, a plunger 200 independently moves backward within a plunger assembly. However, a piston 80 is detached from the plunger 200, thereby causing reuse to be impossible.

### Background Art Documents

(Document 1) Japanese Patent No. 5567073
(Document 2) Japanese Patent No. 5453394
(Document 3) Japanese Patent No. 5712200
(Document 4) Korean Patent No. 10-1541662

### DISCLOSURE

### Technical Problem

Accordingly, the present disclosure has been made in consideration of the above-described problems occurring in the related art, and according to an objective of the present disclosure, a user may slide a plunger to move backward without using a motor.

In addition, according to another objective of the present disclosure, a push rod part may move linearly instead of rotating even when a screw rotates.

Furthermore, according to another objective of the present disclosure, a plunger may be used in a device capable of injecting a medicine such as insulin or hormone so that the medicine may be safely injected during use and the waiting time of the user may be reduced after use.

### Technical Solution

In order to achieve the above objectives, a plunger according to one aspect of the present disclosure may include: a push rod part (10) and the rotation part (20). The push rod part (10) may include: a first housing (110) into which a rotation part (20) is introduced to move linearly; a second housing (120) provided on one end of the first housing (110) and configured to prevent spinning caused by the rotation part (20); a first cover part (130) coupled to a side surface of the second housing (120) and configured to prevent adjustment parts (150) from being detached; a second cover part (140) provided on the other end of the first housing (110); and the one or more adjustment parts (150) provided within the second housing (120), the adjustment parts (150) being configured to be coupled to or decoupled from each other to allow the push rod part (10) to move back and forth. The rotation part (20) may be introduced into the first housing (110) and the second housing (120) to move linearly, and include a rod part (21) having a thread provided on an outer circumferential portion, by which the rotation part (20) is rotatable.

The second housing (120) may include a first protrusion (124) and a second slope (125) provided on a first side portion (123), such that the one or more adjustment parts (150) are coupled to or decoupled from each other.

The second housing (120) may include a first slope (121) and a second slope (122) on inner portions thereof, such that the one or more adjustment parts (150) are decoupled and spaced apart from each other.

The first cover part (130) may include a third protrusion (131) and a fourth protrusion (132) on one surface thereof, such that the one or more adjustment parts (150) are coupled to or decoupled from each other.

Each of the adjustment parts (150) may include: a first flat portion (151) having an inclination to be attached to or detached from the second housing (120); a first recess (152) configured to be coupled to a first protrusion (124) or a fourth protrusion (132), and to be slidable; a second recess (153) configured to be coupled to a second slope (125) or a third protrusion (131), and to be slidable; and a first curved portion (155) including a curved surface provided on an inner portion of each of the adjustment parts (150), with a thread being provided on the curved surface, such that the first curved portion (155) is coupled to the rotation part (20).

Each of the adjustment parts (150) may further include a first curved slope (154) on one side of the first curved portion (155) and a second curved slope (156) on the other side of the first curved portion (155) to reduce friction between the first curved portion (155) and the rotation part (20).

In addition, the plunger according to the present disclosure may further include a power transmission (G) and a power generating device (2) on the other end of the rotation part (20) to automatically drive the rotation part (20).

The plunger according to the present disclosure may be included in a medicine injection device configured to inject one selected from insulin, hormone, heparin, or derivatives thereof.

### Advantageous Effects

In the plunger according to the present disclosure, the push rod part can be moved backward in a short time without use of a motor, thereby allowing a user to conveniently use the plunger.

In addition, according to the present disclosure, a motor may not be noisy when backwardly moving the plunger, and the lifespan of the motor may be increased.

Furthermore, the plunger according to the present disclosure is used in a device configured to inject a medicine, such as insulin or hormone, so that the medicine can be safely injected during use and the waiting time of the user can be reduced after use.

### DESCRIPTION OF DRAWINGS

FIGS. 1 to 4 illustrate background-art Documents of the present disclosure;
FIG. 5 is a perspective view illustrating a plunger according to the present disclosure;
FIG. 6 is an exploded perspective view illustrating the plunger according to the present disclosure;
FIG. 7 is an exploded right side view illustrating the second housing according to the present disclosure;
FIG. 8 is a perspective view illustrating the adjustment part according to the present disclosure;
FIG. 9 is a front view illustrating the second housing, the adjustment part, and the first cover part fitted together according to the present disclosure;
FIG. 10 is a perspective view illustrating the first housing and the second cover part fitted together according to the present disclosure;
FIG. 11 is a perspective view illustrating the first adjusting part and the second adjusting part coupled to each other according to the present disclosure;
FIG. 12 is a perspective view illustrating the first adjusting part and the second adjusting part spaced apart from each other according to the present disclosure;
FIG. 13 is a perspective front view illustrating the first adjusting part and the second adjusting part spaced apart from each other according to the present disclosure;
FIG. 14 is a perspective view illustrating the difference between the coupled distance and the spaced distance between the first adjusting part and the second adjusting part according to the present disclosure; and
FIG. 15 is a view illustrating a situation in which the plunger according to the present disclosure is coupled to a power transmission and a power generator.

### <Description of Reference Numerals of Drawings>

1: plunger; 2: power generating device;
10: push rod part;
20: rotation part; 21: rod part; 22: first detachment prevention part; 23: second detachment prevention part;
110: first housing; 111: first protrusion; 112: second protrusion;
120: second housing; 121: first slope; 122: second slope; 123: first side portion; 124: first protrusion; 125: second protrusion; 126: first coupling recess; 127: second coupling recess; 128: first punched portion;
130: first cover part; 131: third protrusion; 132: fourth protrusion; 133: first coupling portion; 134: second coupling portion 134;
140: second cover part; 141: first fastening portion; 142: second fastening portion; 143: third fastening portion;
150: adjustment part; 150-1: first adjustment part; 150-2: second adjustment part; 151: first flat portion; 152: first recess; 153: second recess; 154: first curved slope; 155: first curved portion; 156: second curved slope;
G: power transmission

### BEST MODE

Hereinafter, embodiments of the present disclosure will be described in detail.

Objectives, features, and advantages of the present disclosure will be apparent from the following embodiments.

The present disclosure should not be construed as being limited to the embodiments to be disclosed herein and may be embodied in a variety of different forms. The following embodiments will be provided for illustrative purposes to fully convey the concept of the present disclosure to those having ordinary knowledge in the technical field, and should be interpreted as including all modifications, equivalents, and substitutions without departing from the technical idea and scope of the present disclosure.

Therefore, the present disclosure is not limited by the following embodiments but should be interpreted as including all modifications without departing from the technical idea and scope of the present disclosure. That is, those having ordinary knowledge in the technical field to which the present disclosure pertains can make various modifications and variations of the present disclosure by adding, changing, deleting, or supplementing elements without departing from the principle of the present disclosure described in the Claims, and such modifications and variations should be included within the scope of the present disclosure.

Although the present disclosure may be modified variously and have a variety of embodiments, specific embodiments will be illustrated in the drawings and described in detail. In the drawings, the sizes of the elements and the relative sizes of the elements may be exaggerated for a better understanding of the present disclosure. In addition, the shapes of the elements illustrated in the drawings may be changed more or less due to changes in fabrication process or the like.

Therefore, the following embodiments should not be limited to the shapes illustrated in the drawings unless expressly so stated herein but should be understood as including some variations.

In the meantime, several embodiments of the present disclosure may be coupled to other embodiments unless explicitly stated to the contrary. In particular, any preferable or advantageous features may be combined with any other feature or features. That is, various aspects, features, embodiments, or implementations of the present disclosure may be used either alone or in various combinations.

It should be understood that each of terms used herein is to describe a specific embodiment but is not to be limited by the Claims. Unless otherwise specified, all terms including technical and scientific terms used herein have the same meaning as that commonly understood by those having ordinary knowledge in the art. As used herein, singular forms are intended to include plural forms unless the context clearly indicates otherwise.

In the description of the present disclosure, detailed descriptions of well-known functions and components incorporated herein will be omitted when it is determined that the description may make the subject matter of the present disclosure rather unclear.

In the present disclosure, forward movement of a plunger 1 indicates that a rotation part 20 moves out of a push rod part 10 in response to the push rod part 10 moving in the direction from a second housing 120 to a second cover part 140.

In addition, in the present disclosure, backward movement of the plunger 1 indicates that the rotation part 20 moves into the push rod part 10 in response to the push rod part 10 moving in the direction from the second cover part 140 to the second housing 120.

In addition, in the present disclosure, the plunger 1 may be formed from one or more selected from metals or synthetic resins. The present disclosure is not limited thereto, since the plunger may be from different materials selected according to the intended use thereof.

A power transmission G in the present disclosure means a wheel having teeth provided on the periphery at regular pitches, i.e., a gear.

### <First Embodiment>

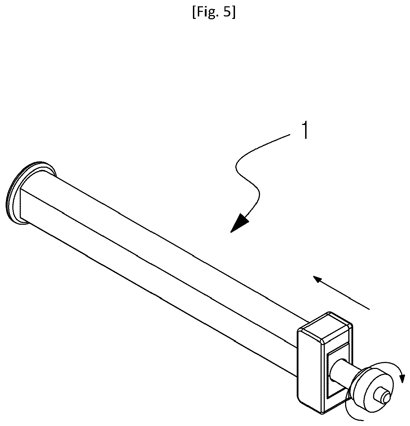

In the present disclosure, as illustrated in FIG. 5, the plunger 1 includes the push rod part 10 to move linearly in the horizontal direction and the rotation part 20 to rotate.

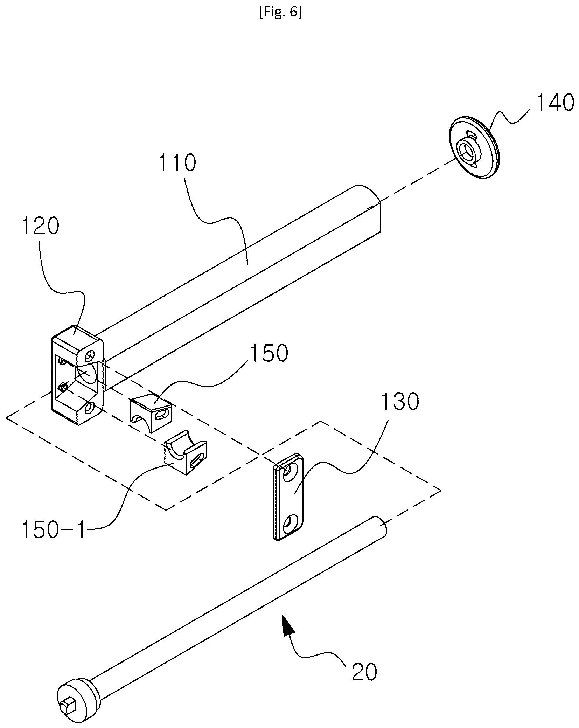

As illustrated in FIG. 6, the push rod part 10 includes a first housing 110, the second housing 120, a first cover part 130, the second cover part 140, and adjustment parts 150.

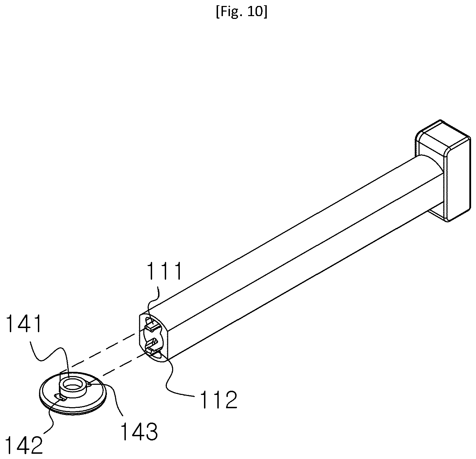

As illustrated in FIGS. 6 and 10, the first housing 110 is a part into which the rotation part 20 is introduced to move linearly. The first housing 110 is a figure obtained by rotating a rectangle once about one side thereof. The first housing 110 may have a shape, the interior of which is penetrated, i.e., have a hollow cylindrical shape, but the present disclosure is not limited thereto.

No threads are provided inside the first housing 110, and the first housing 110 is not configured such that threads of the rotation part 20 are fixedly engaged with the inside of the first housing 110. Thus, the rotation part 20 naturally slides when introduced into the housing 110, and linear movement of the push rod part 10 is not obstructed.

That is, there is an advantage in that no friction occurs between the inside of the first housing 110 and the threads of the rotation part 20.

As illustrated in FIG. 10, the first housing 110 has a first protrusion 111 and a second protrusion 112 provided on the other end thereof in order to be coupled to the second cover part 140.

The first protrusion 111 and the second protrusion 112 protrude from inner portions of the other end of the first housing 110, and stepped portions are formed in the direction of the center of the first housing 110.

The stepped portions provided on the first protrusion 111 and the second protrusion 112 are engaged with catch recesses of a second fastening portion 142 and a third fastening portion 143, such that the second cover part 140 is coupled to the other end of the first housing 110.

In addition, each of the first protrusion 111 and the second protrusion 112 protrudes from the other end of the first housing 110 so as to not have friction with the rotation part 20 being introduced into the first housing 110.

As illustrated in FIG. 6, the second housing 120 is a portion provided on one end of the first housing 110 to prevent the push rod part 10 from being spun by the rotation part 20. The cross-section of the second housing 120 may be a polygon having four sides and four vertices, i.e., a quadrilateral.

When the second housing 120 is provided as a cylinder in the same manner as the first housing 110, the push rod part 10 is rotated instead of being moved linearly by the rotation part 20 rotating as the threads thereof engage with the threads of the adjustment parts 150 coupled to the inside of the second housing 120. In such a situation, the push rod part 10 idles, which is problematic.

That is, particularly, the second housing 120 may have a rectangular cross-section in which opposite sides of two pairs of sides are parallel to each other and have the same lengths in order to prevent such problems.

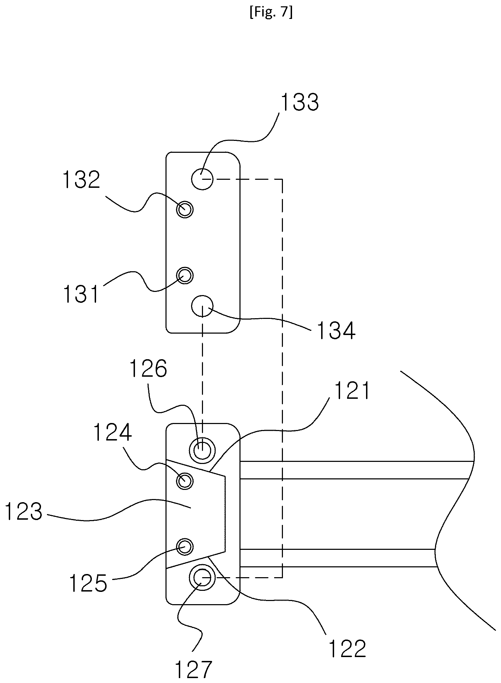

As illustrated in FIG. 7, the second housing 120 includes a first slope 121, a second slope 122, a first side portion 123, a first protrusion 124, a second protrusion 125, a first coupling recess 126, a second coupling recess 127, and a first punched portion 128.

The first slope 121 may have an angle of inclination on one surface inside the second housing 120.

The second slope 122 may have an angle of inclination on the other surface inside the second housing 120, i.e., the surface opposite the first slope 121.

In addition, as illustrated in FIG. 7, the first slope 121 and the second slope 122 are symmetrically inclined, thereby forming a taper, the diameter of which gradually decreases in the direction of the first housing 110.

The inner cross-sectional shape of the second housing 120, to which the adjustment parts 150 are coupled, may be a quadrilateral in which two opposite sides of one pair of sides are parallel to each other and the remaining two non-parallel sides have the same lengths, i.e., an isosceles trapezoid.

The first side portion 123 is a vertical surface between the first slope 121 and the second slope 122.

The first protrusion 124 and the second protrusion 125 may protrude perpendicularly to the first side portion 123, and have a circular cross-section.

Here, the first protrusion 124 and the second protrusion 125 may be provided symmetric about the centerline.

The first coupling recess 126 may be a concave recess having a circular cross-section, provided on one surface of the outer circumferential portion of the second housing 120.

The second coupling recess 127 may be provided on the same surface as the first coupling recess 126, symmetrically about the centerline. The second coupling recess 127 may have the same shape as the first coupling recess 126.

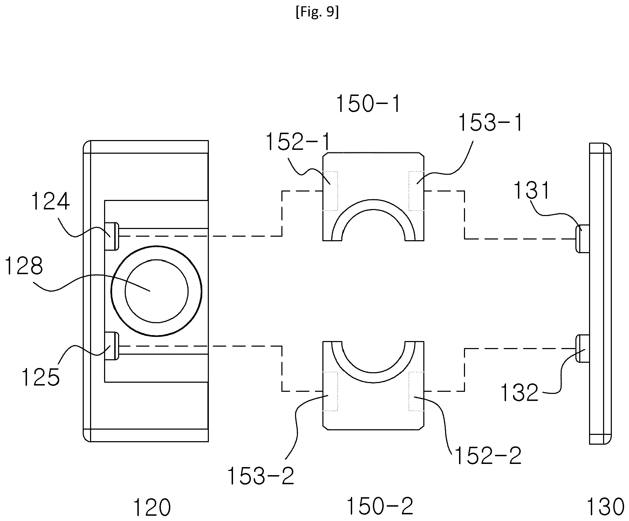

As illustrated in FIG. 9, the first punched portion 128 is punched in the inner surface of the second housing 120 provided on one end of the first housing 110, such that the rotation part 20 is introduced into the first housing 110.

The diameter of the first punched portion 128 may be the same as or greater than the rotation part 20.

When the diameter of the first punched portion 128 is smaller than the diameter of the rotation part 20, the rotation part 20 cannot be introduced into the first housing 110, and thus the plunger function cannot be performed.

As illustrated in FIG. 6, the first cover part 130 is coupled to the side surface of the second housing 120 and prevents the adjustment parts 150 from being detached. The first cover part 130 may have the same cross-section as the second housing 120.

As illustrated in FIG. 7, the first cover part 130 includes a third protrusion 131, a fourth protrusion 132, a first coupling portion 133, and a second coupling portion 134.

The third protrusion 131, the fourth protrusion 132, the first coupling portion 133, and the second coupling portion 134 may be provided on one surface of the first cover part 130 coupled to the second housing 120.

The third protrusion 131 protrudes perpendicularly from one surface of the first cover part 130 to the same height as the opposite first protrusion 124. The third protrusion 131 may have a circular cross-section.

The fourth protrusion 132 protrudes perpendicularly from one surface of the first cover part 130 to the same height as the opposite second protrusion 125. The fourth protrusion 132 may have a circular cross-section.

The first coupling portion 133 protrudes perpendicularly from one surface of the first cover part 130 to the same height as the opposite second coupling recess 127. The first coupling portion 133 may have a circular cross-section.

In addition, the cross-sectional diameter of the first coupling portion 133 is the same as that of the second coupling recess 127. The first coupling portion 133 may be introduced in the direction of the second coupling recess 127 for fixed coupling.

The second coupling portion 134 protrudes perpendicularly from one surface of the first cover part 130 to the same height as the opposite first coupling recess 126. The second coupling portion 134 may have a circular cross-section.

In addition, the cross-sectional diameter of the second coupling portion 134 is the same as that of the first coupling recess 126. The second coupling portion 134 may be introduced in the direction of the first coupling recess 126 for fixed coupling.

That is, the first cover part 130 is configured to be fitted to the second housing 120 like a Lego block. Since the first cover part 130 has an attachable and detachable structure, the adjustment parts 150 are advantageously replaceable.

As illustrated in FIG. 6, the second cover part 140 is provided on the other end of the first housing 110. The second cover part 140 may have a circular cross-section.

In addition, the diameter of the second cover part 140 may be the same as or greater than that of the first housing 110.

As illustrated in FIG. 10, the second cover part 140 includes a first fastening portion 141, the second fastening portion 142, and the third fastening portion 143.

The first fastening portion 141, the second fastening portion 142, and the third fastening portion 143 may be provided on one surface of the second cover part 140 coupled to the first housing 110.

The first fastening portion 141 protrudes perpendicularly from one surface of the second cover part 140. The first fastening portion 141 may be introduced into the housing 110 in the direction of the other end and coupled to a portion of the housing 110 between the first protrusion 111 and the second protrusion 112.

The second fastening portion 142 is provided as a recess on the same surface of the second cover part 140, from which the first fastening portion 141 protrudes. A catch recess is provided inside the second fastening portion 142.

The third fastening portion 143 is provided as a recess on the same surface of the second cover part 140, from which the fastening portion 141 protrudes. The third fastening portion 143 is symmetrical to the second fastening portion 142, and a catch recess is provided inside the third fastening portion 143.

As illustrated in FIG. 10, the second fastening portion 142 is fixed by engaging the catch recess thereof with the above-described stepped portion of the first protrusion 111, and the third fastening portion 143 is fixed by engaging the catch recess thereof with the above-described stepped portion of the second protrusion 112.

That is, as the second fastening portion 142 and the third fastening portion 143 are coupled to the first protrusion 111 and the second protrusion 112, respectively, the fixing force of the second cover part 140 is increased. Thus, when coupled to a medicine injection device (not shown), the second cover part 140 is not detached from the first housing 110 even when the plunger 1 is pushed or pulled.

As illustrated in FIG. 6, one or more adjustment parts 150 are provided inside the second housing 120. The adjustment parts 150 may be coupled or decoupled, thereby allowing the push rod part 10 to move back and forth. The adjustment parts 150 may have a quadrilateral cross-section in which two opposite sides of one pair of sides are parallel to each other, i.e., a trapezoid.

Particularly, the adjustment parts 150 may have a trapezoidal cross-section in which the opposite sides of one pair of sides are parallel to each other, one of the remaining sides is perpendicular to the pair of parallel sides, and the other of the remaining sides is inclined at an angle.

In addition, as illustrated in FIG. 9, two adjustment parts 150 are included inside the second housing 120 and coupled to each other to be symmetrical about the centerline. Thus, one adjustment part 150 in contact with the inner top surface of the second housing 120 is the first adjustment part 150-1, while the other adjustment part 150 in contact with the inner bottom surface of the second housing 120 is the second adjustment part 150-2.

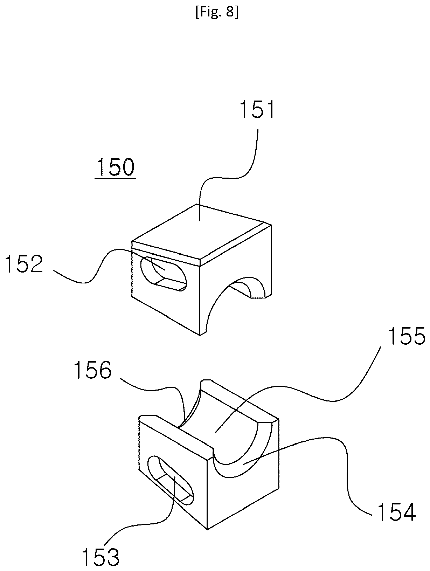

As illustrated in FIG. 8, the adjustment parts 150 respectively include first flat portion 151, a first recess 152, a second recess 153, a first curved slope 154, a first curved portion 155, and a second curved slope 156.

As illustrated in FIG. 8, the first flat portion 151 corresponds to the top surface, and is provided as an inclined surface to be in contact with and attached to or detached from the first slope 121 or the second slope 122 provided on the inner portion of the second housing 120.

In addition, the angle of inclination of the first flat portion 151 may be the same as or smaller than the angle of inclination of the first slope 121 or the second slope 122 provided inside the second housing 120.

Thus, when the adjustment parts 150 are decoupled into the first adjustment part 150-1 and the second adjustment part 150-2 within the second housing 120, the adjustment parts 150 may naturally slide with no friction and portions of the adjustment parts 150 may move from the inside of the second housing 120.

The first recess 152 may be an elliptical recess provided on one outer surface of the adjustment part 150. Particularly, the first recess 152 may have an elliptical shape in which a rectangle is positioned between and combined with two semicircles having the same diameter.

As illustrated in FIG. 9, in a situation in which the first protrusion 124 or the second protrusion 125 protruding from the second housing 120 or the third protrusion 131 or the fourth protrusion 132 protruding from the first cover part 130 is coupled to the first recess 152, even in the case of sliding, movement may be performed by a predetermined distance, and disengagement may be prevented.

The second recess 153 may be provided on the other outer surface of the adjustment part 150. The second recess 153 may be parallel to and have the same shape as the first recess 152 to provide the same function as the first recess 152.

More specifically, as illustrated in FIG. 9, the first protrusion 124 protruding from the second housing 120 is fitted into and coupled to the first recess 152-1 of the first adjustment part 150-1, and the fourth protrusion 132 protruding from the first cover part 130 is fitted into and coupled to the first recess 152-2 of the second adjustment part 150-2.

In addition, as illustrated in FIG. 9, the third protrusion 131 protruding from the first cover part 130 is fitted into and coupled to the second recess 153-1 of the first adjustment part 150-1, and the second protrusion 125 of the second housing 120 is fitted into and coupled to the second recess 153-2 of the second adjustment part 150-2.

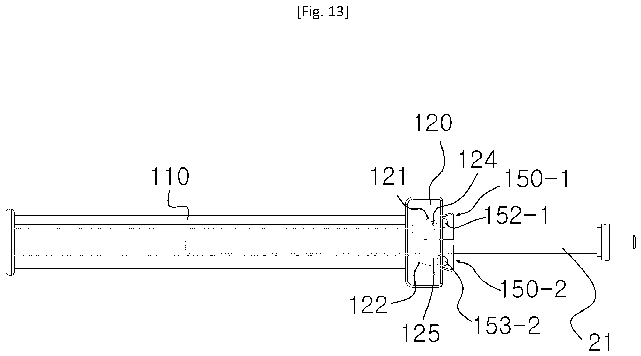

That is, as illustrated in FIG. 13, in the adjustment parts 150, in a situation in which the first protrusion 124 and the second protrusion 125 protruding from the second housing 120 and the third protrusion 131 and the fourth protrusion 132 protruding from the first cover part 130 are fixed, the first recess 152 and the second recess 153 recessed in the adjustment parts 150 slide in the direction from the first housing 110 to the second housing 120 or from the second housing 120 to the first housing 110. Consequently, the rotation part 20 may freely move back and forth.

In FIG. 8, in a position in which the first flat portion 151 is the top surface, the first curved slope 154 is provided on the bottom of the right side of the adjustment part 150, in the shape of a semicircle having an inclined recess. Thus, the first curved slope 154 may reduce friction between the first curved portion 155 and the rotation part 20.

The first curved portion 155 is provided on the bottom surface of the adjustment part 150 as a semi-cylindrical recess including a female thread.

In addition, the length of the radius of the semicircle of the first curved portion 155 is the same as the length of the radius of the rotation part 20. As the female thread engages with the male thread of the rotation part 20, the push rod part 10 may move linearly in the horizontal direction.

In FIG. 8, in a position in which the first flat portion 151 is the top surface, the second curved slope 156 is provided on the bottom of the left side of the adjustment part 150, in the shape of a semicircle having an inclined recess. Thus, the second curved slope 156 may reduce friction between the first curved portion 155 and the rotation part 20.

In the present disclosure, in the adjustment parts 150, the first curved portion 155-1 of the first adjustment part 150-1 and the first curved portion 155-2 of the second adjustment part 150-2 may adjoin each other, thereby forming a circular shape.

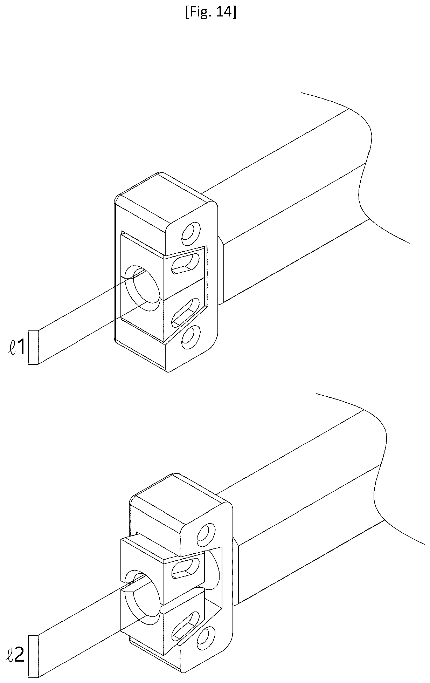

As illustrated in FIG. 14, ℓ1 is the distance between the first curved portion 155-1 of the first adjustment part 150-1 and the first curved portion 155-2 of the second adjustment part 150-2 when the first curved portion 155-1 and the first curved portion 155-2 adjoin each other.

ℓ2 is the distance between the first curved portion 155-1 of the first adjustment part 150-1 and the first curved portion 155-2 of the second adjustment part 150-2 when the first curved portion 155-1 and the first curved portion 155-2 are spread apart in response to the first adjustment part 150-1 and the second adjustment part 150-2 being spaced apart from each other as the first recess 152 and the second recess 153 of the first adjustment part 150-1 and the second adjustment part 150-2 that have been fitted around and coupled to the first protrusion 124 and the second protrusion 125 protruding from the second housing 120 and the third protrusion 131 and the fourth protrusion 132 protruding from the first cover part 130 slide in the direction from the first housing 110 to the second housing 120.

The distance ℓ1 should be set to be the same as the length of the diameter of a rod part 21 to be described later. When the distance ℓ1 is smaller than the length of the diameter of the rod part 21, the plunger 1 cannot be driven.

In addition, when the distance ℓ1 is greater than the length of the diameter of the rod part 21, when the rotation part 20 is rotated, the rotation part 20 spins between the first adjustment part 150-1 and the second adjustment part 150-2. Consequently, it is difficult to accurately realize linear movement of the rod part 10.

Furthermore, ℓ1 should be set to be smaller than ℓ2. In this case, when the first adjustment part 150-1 and the second adjustment part 150-2 are spaced apart from each other, the user can advantageously move the plunger 1 backward by pushing the rod part 10 in the direction of the rotation part 20 or by using gravity by inverting the device without using a motor.

Since a plunger of the related art uses a pressure gear in the rotation part, a long time takes to return to the original position. Thus, there have been problems in terms of waiting time and reduced lifespan.

That is, the present disclosure uses the adjustment parts 150 in order to overcome such problems. Thus, the plunger 1 takes 1 or 2 seconds to return to the original position, thereby advantageously reducing the waiting time of the user.

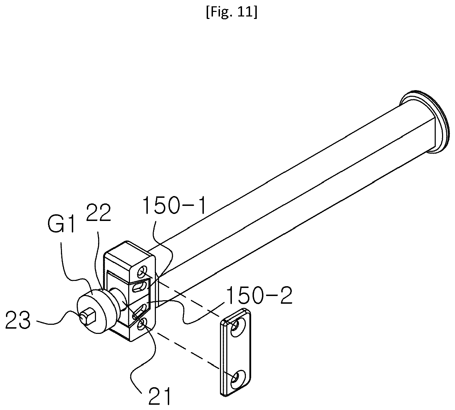

As illustrated in FIG. 11, the rotation part 20 includes the rod part 21, a first detachment prevention part 22, a power transmission G, and a second detachment prevention part 23.

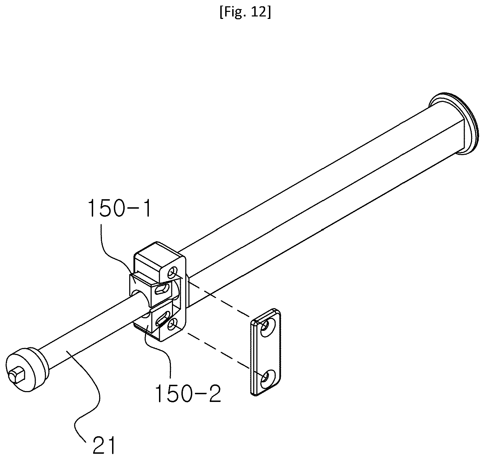

As illustrated in FIG. 12, the rod part 21 is introduced into the first housing 110 and the second housing 120 so that the push rod part 10 is moved linearly. The rod part 21 has the male thread on the outer circumferential portion. The male thread of the rod part 21 engages with the female threads of the first adjustment part 150-1 and the second adjustment part 150-2, thereby rotating the rod part 21.

In addition, the rod part 21 may be a figure obtained by rotating a rectangle once about one side thereof, i.e., a cylindrical shape.

The diameter of the rod part 21 is set to be the same as the diameter length ℓ1, and the length of the rod part 21 is set to be smaller than the length of the first housing 110.

The first detachment prevention part 22 is provided on one end surface of the rod part 21 to prevent a first power transmission G1 from being detached.

In addition, when the first adjustment part 150-1 and the second adjustment part 150-2 that have been spaced apart from each other return to the original positions, the first detachment prevention part 22 is in contact with the portion on which the first curved slope 154 is formed, and the first adjustment part 150-1 and the second adjustment part 150-2 are introduced in the direction from the second housing 120 to the first housing 110. Consequently, the first adjustment part 150-1 and the second adjustment part 150-2 return to the coupled positions.

The first power transmission G1 is a gear. As illustrated in FIG. 11, the first power transmission G1 is provided between the first detachment prevention part 22 and the second detachment prevention part 23.

The second detachment prevention part 23 is provided on one end of the rod part 21, and prevents the first power transmission G1 from being detached.

### MODE FOR INVENTION

### <Second Embodiment>

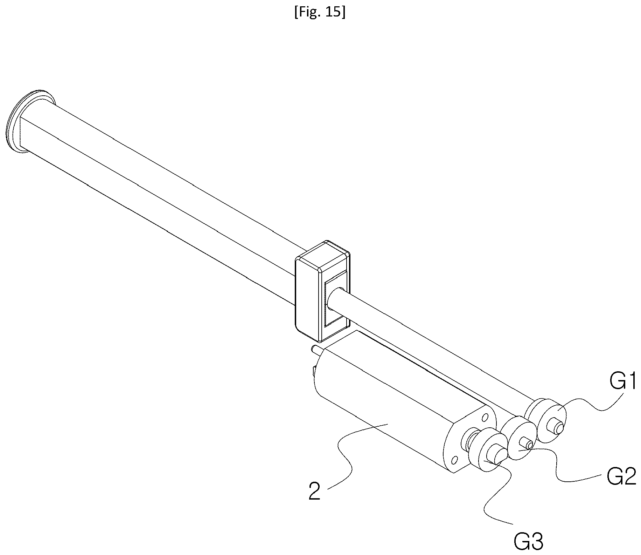

As illustrated in FIG. 15, a second power transmission G2, a third power transmission G3, and a power generating device 2 may be included in order to automatically drive the rotation part 20 according to the first embodiment.

The plunger 1 according to the first embodiment has the same configuration, and thus a description thereof will be omitted, and the remaining components will be described.

As illustrated in FIG. 15, the second power transmission G2 is provided between the third power transmission G3 coupled to one end of the power generating device 2 and the first power transmission G1 provided on one end surface of the rotation part 20.

The second power transmission G2 is a gear positioned between the first power transmission G1 and the third power transmission G3 to rotate by engagement of teeth of the second power transmission G2 with teeth of the first power transmission G1 and teeth of the third power transmission G3. Thus, no slip may occur, and energy generated by the power generating device 2 may be transferred to the first power transmission G1 without a loss, thereby rotating the rotation part 20.

The third power transmission G3 is a gear provided on one end of the power generating device 2. The teeth of the third power transmission G3 are engaged with the teeth of the second power transmission G2, so that the third power transmission G3 transfers energy generated by the power generating device 2 to the second power transmission G2.

The power generating device 2 may be implemented as a motor, i.e., a power machine that rotates by receiving electric power and generates rotational force about the axis thereof.

In the present disclosure, the power transmission G and the power generating device 2 disclosed herein are known in the art.

Since the plunger 1 according to the present disclosure includes the power generating device 2, in a position as illustrated in FIG. 11, when the rotation part 20 is rotated by the power generating device 2, the push rod part 10 moves linearly in the direction from the second housing 120 to the first housing 110.

In addition, when the rotation part 20 does not rotate any more, the first flat portions 151-1 and 151-2 of the first adjustment part 150-1 and the second adjustment part 150-2 are slid in the direction of inclination, i.e., in the direction from the second housing 120 to the rotation part 20, along the first slope 121 and the second slope 122, respectively, as illustrated in FIG. 13. Consequently, the first adjustment part 150-1 and the second adjustment part 150-2 are spaced apart from each other.

As illustrated in FIGS. 13 and 14, the spaced length ℓ2 between the first adjustment part 150-1 and the second adjustment part 150-2 is set to be greater than the diameter of the rod part 21, so that the push rod part 10 can move backward, i.e., in the direction of the rotation part 20.

Afterwards, when the portions on which the first curved slopes 154-1 and 154-2 of the first adjustment part 150-1 and the second adjustment part 150-2 are formed are in contact with the first detachment prevention part 22, respectively, the first flat portions 151-1 and 151-2 of the first adjustment part 150-1 and the second adjustment part 150-2 are slid in the direction of the second housing 120 to the first housing 110 along the first slope 121 and the second slope 122, respectively. Consequently, the first adjustment part 150-1 and the second adjustment part 150-2 are in contact with each other.

Here, the female threads provided on the first adjustment part 150-1 and the second adjustment part 150-2, respectively, engage with the male thread provided on the rod part 21.

### <Third Embodiment>

The medicine injection device includes the plunger 1 according to the foregoing first or second embodiment.

The medicine injection device refers to a device designed to inject a medicine. For example, a syringe or an automatic syringe may be used. The automatic syringe may be one of a pump type syringe or a pen type syringe.

The medicine may include one or more selected from, but is not limited to, insulin, hormone, low-molecular-weight heparin, or derivatives thereof.

In addition, the medicine injection device refers to a disposable or reusable device configured to inject medicine once or several times.

That is, the use of the plunger 1 according to the present disclosure makes it possible to safely inject a predetermined dose of medicine into the human body using the power generating device 2. After the injection of the medicine is completed, the adjustment parts 150 are decoupled into the first adjustment part 150-1 and the second adjustment part 150-2, so that the plunger 1 is moved backward without the use of the power generating device 2. Advantageously, the waiting time of the user is significantly reduced.

In addition, a method of decoupling the adjustment parts 150 so that the plunger 1 coupled to the medicine injection device moves backward is to remove a medicine cartridge.

Afterwards, when the user pulls the push rod part 10 with a hand, the first recess 152 and the second recess 153, fitted into and coupled to the first protrusion 124, the second protrusion 125, the third protrusion 126, and the fourth protrusion 127, are slid along the first slope 121 and the second slope 122, respectively, so that the first adjustment part 150-1 and the second adjustment part 150-2 are spaced apart from each other. Consequently, the male thread of the rod part 21 is disengaged from the female threads of the first adjustment part 150-1 and the second adjustment part 150-2.

Another method of decoupling the adjustment parts 150 so that the plunger 1 coupled to the medicine injection device moves backward is to move the power generating device 2 by a predetermined length in the reverse direction by executing a cartridge replacement menu after the injection of the medicine is completed.

Here, the first recess 152 and the second recess 153, fitted into and coupled to the first protrusion 124, the second protrusion 125, the third protrusion 126, and the fourth protrusion 127, are slid by predetermined lengths along the first slope 121 and the second slope 122, respectively, so that the first adjustment part 150-1 and the second adjustment part 150-2 are spaced apart from each other. Consequently, the male thread of the rod part 21 is disengaged from the female threads of the first adjustment part 150-1 and the second adjustment part 150-2.

### INDUSTRIAL APPLICABILITY

The present disclosure relates to a plunger movable back and forth and a medicine injection device including the same, wherein the plunger can be manually or automatically moved backward to reduce the waiting time of a user. Accordingly, the present disclosure is industrially applicable.

## Claims

1. A plunger comprising:
a push rod part (10) comprising:
- a first housing (110) into which a rotation part (20) is introduced to move linearly;
- a second housing (120) provided on one end of the first housing (110) and configured to prevent spinning caused by the rotation part (20);
- a first cover part (130) coupled to a side surface of the second housing (120) and configured to prevent adjustment parts (150) from being detached;
- a second cover part (140) provided on the other end of the first housing (110); and
- the one or more adjustment parts (150) provided within the second housing (120), the adjustment parts (150) being configured to be coupled to or decoupled from each other to allow the push rod part (10) to move back and forth; and
the rotation part (20) introduced into the first housing (110) and the second housing (120) to move linearly, and comprising a rod part (21) having a thread provided on an outer circumferential portion, by which the rotation part (20) is rotatable.

2. The plunger of claim 1, wherein the second housing (120) comprises a first protrusion (124) and a second protrusion (125) provided on a first side portion (123), such that the one or more adjustment parts (150) are coupled to or decoupled from each other.

3. The plunger of claim 1, wherein the second housing (120) comprises a first slope (121) and a second slope (122) on inner portions thereof, such that the one or more adjustment parts (150) are decoupled and spaced apart from each other.

4. The plunger of claim 1, wherein the first cover part (130) comprises a third protrusion (131) and a fourth protrusion (132) on one surface thereof, such that the one or more adjustment parts (150) are coupled to or decoupled from each other.

5. The plunger of claim 1, wherein each of the adjustment parts (150) comprises:
a first flat portion (151) having an inclination to be attached to or detached from the second housing (120);
a first recess (152) configured to be coupled to a first protrusion (124) or a fourth protrusion (132), and to be slidable;
a second recess (153) configured to be coupled to a second protrusion (125) or a third protrusion (131), and to be slidable; and
a first curved portion (155) comprising a curved surface provided on an inner portion of each of the adjustment parts (150), with a thread being provided on the curved surface, such that the first curved portion (155) is coupled to the rotation part (20).

6. The plunger of claim 5, wherein each of the adjustment parts (150) further comprises a first curved slope (154) on one side of the first curved portion (155) and a second curved slope (156) on the other side of the first curved portion (155) to reduce friction between the first curved portion (155) and the rotation part (20).

7. The plunger of claim 1, further comprising a power transmission (G) and a power generating device (2) on the other end of the rotation part (20) to automatically drive the rotation part (20).

8. A medicine injection device comprising a plunger as claimed in any one of claims 1 to 7.
